(19) Europäisches Patentamt
European Patent Office
Office européen des brevets

(11) EP 1 280 790 B1

(12) **EUROPÄISCHE PATENTSCHRIFT**

(45) Veröffentlichungstag und Bekanntmachung des Hinweises auf die Patenterteilung:
**10.12.2003 Patentblatt 2003/50**

(21) Anmeldenummer: **01929610.2**

(22) Anmeldetag: **28.04.2001**

(51) Int Cl.[7]: **C07D 317/14**, C07D 319/06

(86) Internationale Anmeldenummer:
**PCT/EP01/04821**

(87) Internationale Veröffentlichungsnummer:
**WO 01/085713 (15.11.2001 Gazette 2001/46)**

(54) **CYCLISCHE KETALE ALS DUFTSTOFFE**

USE OF CYCLIC KETALS AS A FRAGRANCE

CETALS CYCLIQUES ET LEUR UTILISATION COMME SUBSTANCES ODORIFERANTES

(84) Benannte Vertragsstaaten:
**AT BE CH CY DE DK ES FI FR GB GR IE IT LI LU MC NL PT SE TR**

(30) Priorität: **09.05.2000 DE 10022417**

(43) Veröffentlichungstag der Anmeldung:
**05.02.2003 Patentblatt 2003/06**

(73) Patentinhaber: **Kao Corporation**
**Chuo-Ku Tokyo 103-8210 (JP)**

(72) Erfinder:
- **MARKERT, Thomas**
**40789 Monheim (DE)**
- **FABER, Werner**
**47877 Willich (DE)**
- **PIERIK, Theo, ten**
**NL-05916 LE Venlo (NL)**

(74) Vertreter: **Wolf, Matthias**
**Hoffmann - Eitle**
**Patent- und Rechtsanwälte**
**Postfach 81 04 20**
**81904 München (DE)**

(56) Entgegenhaltungen:
**EP-A- 0 276 998**
**DE-A- 19 729 840**
**US-A- 4 198 323**

- **DATABASE SPECINFO [Online] U.S. DoC; CAS Registry No. 74752-98-0, 1995 XP002173658**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Gebiet der Erfindung

Die vorliegende Erfindung betrifft neue cyclische Ketale spezieller Struktur, sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthetischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.

Aus DE-A-197 14 041 sind spezielle Phenonketale bekannt, die blumige, Anthranilat-, Ylang- und Tuberose-Noten aufweisen und die als Riechstoffe eingesetzt werden können. Strukturell handelt es sich dabei - im Gegensatz zu den Verbindungen der vorliegenden Anmeldung - um offenkettige Ketale.

### Beschreibung der Erfindung

Es wurde gefunden, dass die Verbindungen der allgemeinen Formel (I) die oben genannten Forderungen in jeder Hinsicht ausgezeichnet erfüllen und in vorteilhafter Weise als Riechstoffe mit unterschiedlichen nuancierten Geruchsnoten mit guter Haftfestigkeit eingesetzt werden können.

Gegenstand der vorliegenden Erfindung sind zunächst cyclische Ketale der allgemeinen Struktur (I)

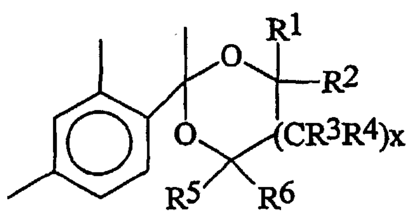

(I)

worin die Reste $R^1$ bis $R^6$ unabhängig voneinander Wasserstoff, Methyl, Ethyl, n-Propyl oder i-Propyl und x die Zahl 0 oder 1 bedeuten. Es sei ausdrücklich festgestellt, dass dann, wenn der Index x die Zahl 0 bedeutet, die beiden Kohlenstoffatome, die die Reste $R^1$ und $R^2$ sowie $R^5$ und $R^6$ tragen, direkt miteinander verknüpft sind.

In einer bevorzugten Ausführungsform der Erfindung haben die Reste $R^1$ bis $R^6$ - unabhängig voneinander - in der allgemeinen Struktur (I) die Bedeutung Wasserstoff oder Methyl.

In einer weiteren Ausführungsform betrifft die Erfindung die Verwendung von cyclischen Ketalen der oben näher bezeichneten allgemeinen Struktur (I) als Riechstoffe. Dabei sind wiederum diejenigen Verbindungen (I) bevorzugt, bei denen die Reste $R^1$ bis $R^6$- unabhängig voneinander- Wasserstoff oder Methyl bedeuten.

Die erfindungsgemäßen Verbindungen (I) zeichnen sich durch eine Geruchscharakteristik aus, in der Orangenblüte-Noten sowie fruchtige und holzige Aspekte dominieren. Sie weisen eine ausgezeichnete Stabilität in Rezepturen der Kosmetik und Gebrauchsparfümerie auf.

Die Herstellung der Verbindungen (I) kann nach bekannten Syntheseverfahren der organischen Chemie erfolgen.

In Parfüm-Kompositionen verstärken die Verbindungen (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindungen (I) die oben genannten Duftnoten aufweisen, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Die Verbindungen der Formel (I) eignen sich aufgrund ihres Geruchsprofiles insbesondere auch zur Modifizierung und Verstärkung bekannter Kompositionen. Hervorgehoben werden soll insbesondere ihre ausserordentliche Geruchsstärke, die ganz allgemein zur Veredelung der Komposition beiträgt.

Die Verbindungen der Formel (I) lassen sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindungen (I) kombiniert werden können sind insbesondere:

(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl,Myrrheöl, Olibanumöl
(b) Alkohole wie Famesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional$^R$, $\alpha$-Hexylzimtaldehyd, Hydroxycitronellal, Lilial$^R$ [p-tert.-Butyl-$\alpha$-methyldihydrozimtaldehyd], Methylnonylacetaldehyd,
(d) Ketone wie Allylionon, $\alpha$-Ionon, $\beta$-Ionon, Isoraldein, Methylionon, Muscon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat, Isobornylacetat,
(f) Lactone wie gamma-Undecalacton, 1-Oxaspiro[4.4]nonan-2-on, sowie verschiedene weitere in der Parfümerie oft benutzte Komponenten wie Moschus- und Sandelholz-Riechstoffe, Indol, p-Menthan-8-thiol-3-on, Methyleugenol und Ambroxan.

Bemerkenswert ist ferner die Art und Weise, wie die Verbindungen der Struktur (I) die Geruchsnoten einer breiten Palette bekannter Kompositionen abrunden und harmonisieren, ohne jedoch in unangenehmer Weise zu dominieren. 2,4-Dimethyl-2-(2,4-dimethylphenyl)-1,3-dioxolan ist in dieser Hinsicht ganz besonders hervorzuheben.

Die einsetzbaren Anteile der erfindungsgemäßen Verbindungen (I) oder deren Gemische in Riechstoffkompositionen bewegen sich von etwa 1-70 Gew. %, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 - 2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen noch Effekte erzielen oder mit noch höheren Dosierungen neuartige Komplexe aufbauen kann.

**Beispiele**

**Beispiel 1:** 2-Methyl-2-(2,4-dimethylphenyl)-1,3-dioxolan

Ansatz:

a) 74,1 g (0,5 mol) 2,4-Dimethylacetophenon (Fa. Fluka)

b) 31,8 g (0,51 mol) Ethylenglykol (1,2 Ethandiol)

c) 0,5 g p-Toluolsulfonsäuremonohydrat (Fa. Fluka)

d) 150 ml Toluol

In einem 0,5 1 Vierhalskolben mit Rührer, Thermometer und Wasserabscheider wurden die 4 Komponenten a) bis d) nacheinander eingetragen und unter Rühren auf Rückflußtemperatur (122°C) gebracht und 27 Stunden lang bei dieser Temperatur gerührt. Danach hatten sich 10 ml Wasser abgeschieden. Die Reaktionsmischung wurde abgekühlt, neutral gewaschen, über $MgSO_4$ getrocknet, am Rotavaporisator eingeengt und der Rückstand an einer 30-cm-Füllkörperkolonne destilliert. Es wurden 79 g Destillat erhalten, die Hauptmenge hatte eine gaschromatographisch (GC) bestimmte Reinheit von 100 %, bei einem Siedepunkt von 79 - 82°C/0.14 mbar. Das $^1$H-NMR-Spektrum zeigte 3 Singuletts (je 1 $CH_3$-Gruppe) bei chemischen Verschiebungen von 1,7; 2,3 und 2,5 ppm, 2 $CH_2$-Gruppen als symmetrische Multipletts bei 3,7 und 4,0 ppm und aromatischen Protonen bei 6.9 (2H) und 7,4 (d, 1H) ppm. Das IR-Spektrum zeigte starke Etherbanden bei 1038 und 1193 und Benzolfinger bei 822 und 866 $cm^{-1}$.

Geruchscharakteristik: Im Angeruch blumig, Teer, Leder, Eichenmoos, Naphthalin, im Nachgeruch krautig, Anthranilat-Note

**Beipiel 2:** 2,4-Dimethyl-2-(2,4-dimethylphenyl)-1,3-dioxolan

Ansatz:

a) 74,0 g (0,5 mol) 2,4-Dimethylacetophenon (Fa. ABCR)

b) 41,9 g (0,55 mol) 1,2 Propandiol (Fa. Merck)

e) 0,5 g p-Toluolsulfonsäuremonohydrat (Fa. Fluka)

f) 150 ml Toluol

In einem 0,5 1 Vierhalskolben mit Rührer, Thermometer und Wasserabscheider wurden die 4 Komponenten a), b), e) und f) nacheinander eingetragen und unter Rühren auf Rückflußtemperatur (130°C) gebracht und 6 Stunden lang bei dieser Temperatur gerührt. Danach hatten sich 10 ml Wasser abgeschieden. Die Reaktionsmischung wurde abgekühlt, neutral gewaschen, über $MgSO_4$ getrocknet, am Rotavaporisator eingeengt und 121 g Rückstand an einer 30cm-Füllkörperkolonne destilliert. Es wurden 79 g Destillat erhalten, die Hauptmenge hatte eine gaschromatographisch bestimmte Reinheit von 99% (zwei Peakflächen a'63% + 36%) bei einem Siedepunkt von 84 - 85°C/0.14 mbar. Das $^1$H-NMR-Spektrum zeigte 3 Singuletts (2 $CH_3$-Gruppen) bei chemischen Verschiebungen von 2,3 und 2,5 ppm, 3 Dubletts bei 1,2 und 1,4 (1 $CH_3$-Gruppe) und 1,7 (1 $CH_3$-Gruppe) ppm sowie zwischen 3,3 und 4,4 ppm mehrere Tripletts und Multipletts (3 Hs) und aromatische Protonen bei 6.9 (2H) und 7,4 (d, 1H) ppm. Bei dem Umsetzungsprodukt handelte es sich um die Mischung zweier Isomeren, die im Verhältnis 2:1 anfallen. Das IR-Spektrum zeigte breite Etherbanden bei 936, 952, 1038, 1063, 1084, 1152, 1193 und 1239 $cm^{-1}$.

Geruchscharakteristik: Im Angeruch stark fruchtig, Cassis-, Nitromoschus-Note, im Nachgeruch Anthranilat-, Salicylat-, Rauch-Note.

**Beispiel 3:** 2-Methyl-2-(2,4-dimethylphenyl)-1,3-dioxan

Ansatz:

a) 74,0 g (0,5 mol) 2,4-Dimethylacetophenon (Fa. ABCR)

b) 41,8 g (0,55 mol) 1,3 Propandiol (Fa. Merck)

g) 0,5 g p-Toluolsulfonsäuremonohydrat (Fa. Fluka)

h) 150 ml Toluol

In einem 0,5 1 Vierhalskolben mit Rührer, Thermometer und Wasserabscheider wurden die 4 Komponenten a), b), g) und h) nacheinander eingetragen und unter Rühren auf Rückflußtemperatur (125°C) gebracht und 24 Stunden lang bei dieser Temperatur gerührt. Danach hatten sich 12,5 ml Wasser abgeschieden. Die Reaktionsmischung wurde abgekühlt, neutral gewaschen, über $MgSO_4$ getrocknet, am Rotavaporisator eingeengt und 90 g Rückstand an einer 30cm-Füllkörperkolonne destilliert. Es wurden 57 g Destillat erhalten, die Hauptmenge hatte eine GC-Reinheit von 97%, bei einem Siedepunkt von 90-94°C/0.12 mbar. Das $^1$H-NMR-Spektrum zeigte 3 Singuletts (3 $CH_3$-Gruppen) bei chemischen Verschiebungen von 1,5; 2,3 und 2,4 und bei 3,8 Multipletts (3 $CH_2$-Gruppen) und aromatische Protonen bei 7.0 (2H) und 7,35 (d, 1H) ppm. Das IR-Spektrum zeigte im Bereich zwischen 900 und 1200 $cm^{-1}$ 8 scharfe Banden.

Geruchscharakteristik: im Angeruch blumig, metallisch, Rhabarber-Note im Nachgeruch schwach nach Anthranilat.

**Beispiel 4:** 2,5,5-Trimethyl-2-(2,4-dimethylphenyl)-1,3-dioxan

Ansatz:

a) 74,0 g (0,5 mol) 2,4-Dimethylacetophenon (Fa. ABCR)

b) 57,2 g (0,55 mol) Neopentylglykol (Fa. Cognis)

i) 0,5 g p-Toluolsulfonsäuremonohydrat (Fa. Fluka)

j) 150 ml Toluol

In einem 0,5 1 Vierhalskolben mit Rührer, Thermometer und Wasserabscheider wurden die 4 Komponenten a), b), i) und j) nacheinander eingetragen und unter Rühren auf Rückflußtemperatur (130°C) gebracht und 24 Stunden lang bei dieser Temperatur gerührt. Danach hatten sich 5 ml Wasser abgeschieden. Die Reaktionsmischung wurde abgekühlt, neutral gewaschen, über $MgSO_4$ getrocknet, am Rotavaporisator eingeengt und 126 g Rückstand an einer 30cm-Füllkörperkolonne destilliert. Es wurden 77 g Destillat erhalten, die Hauptmenge hatte eine GC-Reinheit von 100%, bei einem Siedepunkt von 104-105°C/0.15 mbar. Das $^1$H-NMR-Spektrum zeigte 5 Singuletts (5 $CH_3$-Gruppen) bei chemischen Verschiebungen von 0,6; 1,3; 1,5; 2,3 und 2,4 und bei 3,4 1 Quadruplett (2 $CH_2$-Gruppen) und aromatische Protonen bei 7.0 (2H) und 7,35 (d, 1H) ppm. Das IR-Spektrum zeigte scharfe Etherbanden bei 1082 und 1179 $cm^{-1}$.

Geruchscharakteristik: im Angeruch fruchtig, Cassis-, Eukalyptus-, Jonon-Note im Nachgeruch schwach nach Eukalyptus.

**Beispiel 5:** 2,5-Dimethyl-5-propyl-2-(2,4-dimethylphenyl)-1,3-dioxan

Ansatz:

a) 74,0 g (0,5 mol) 2,4-Dimethylacetophenon (Fa. ABCR)

b) 72,6 g (0,55 mol) 2-Methyl-2-propyl-1,3-propandiol (Fa. Cognis)

k) 0,5 g p-Toluolsulfonsäuremonohydrat (Fa. Fluka)

1) 150 ml Toluol

In einem 0,5 1 Vierhalskolben mit Rührer, Thermometer und Wasserabscheider wurden die 4 Komponenten a), b), k) und 1) nacheinander eingetragen und unter Rühren auf Rückflußtemperatur (130°C) gebracht und 5 Stunden lang bei dieser Temperatur gerührt. Danach hatten sich 7,5 ml Wasser abgeschieden. Die Reaktionsmischung wurde abgekühlt, neutral gewaschen, über $MgSO_4$ getrocknet, am Rotavaporisator eingeengt und 141,6 g Rückstand an einer 30cm-Füllkörperkolonne destilliert. Es wurden 77 g Destillat erhalten, die Hauptmenge hatte eine gaschromatographisch bestimmte Reinheit von 96,9% (zwei Peakflächen a' 52,6% + 44,3%) bei einem Siedepunkt von 100-105°C/ 0.07 mbar. Das $^1H$-NMR-Spektrum zeigte 5 Singuletts (4 $CH_3$-Gruppen) bei chemischen Verschiebungen von 0,5; 1,3; 1,5; 2,3 und 2,4 ppm, 2 Tripletts bei 0,8 und 1,0 ppm (1CH3-Gruppe), und Multipletts bei 0,9; 1,1; 1,3 und 1,7ppm (2 $CH_2$-Gruppen) und bei 3,4 ppm 1 Quadruplett und 1 Singulett (2 $CH_2$-Gruppen) und aromatische Protonen bei 7.0 (2H) und 7,3 (d, 1H) ppm. Das IR-Spektrum zeigte 1 scharfe Etherbande bei 1179 $cm^{-1}$.

Geruchscharakteristik: im Angeruch grün, Rosmarin-, Citrusal-Note im Nachgeruch staubig, animalisch, Pferdestall-Note.

**Beispiel 6:** Fruchtige Komposition für den Einsatz in Schampon:

| | |
|---|---|
| Jasmacyclat (Cognis) | 40 Teile |
| Phenirate (H&R) | 5 Teile |
| Geraniol | 15 Teile |
| Floramat (Cognis) | 20 Teile |
| alpha-Hexylzimtaldehyd | 20 Teile |
| Rosenoxid L (Dragoco) | 10 Teile |
| Herbavert (Cognis) | 30 Teile |
| Cyclovertal (Cognis) | 10 Teile |
| Methyldihydrojasmonat | 200 Teile |
| Iraldein gamma (H&R) | 30 Teile |
| Boisambrene forte (Cognis) | 60 Teile |
| Vanillin 10%ig in DPG | 40 Teile |
| Coumarin | 40 Teile |
| Iso E super (IFF) | 20 Teile |
| Sandelice (Cognis) | 210 Teile |
| Astratone | 120 Teile |
| 2,4-Dimethyl-2-(2,4-dimethylphenyl)-1,3-dioxolan (gemäß Beispiel 2) bzw. DPG | 50 Teile |
| Rhuboflor (Firmenich) | 30 Teile |
| | 950,0 Teile |

Der Zusatz von 50 Teilen der Verbindung gemäß Beispiel 2 verstärkt den Fruchtcharakter und verleiht der Komposition einen einzigartigen Charakter nach schwarzen Johannisbeeren. Eine Komposition, in der DPG (Dipropylenglykol) anstatt der Verbindung gemäß Beispiel 2 enthalten ist, präsentiert sich vergleichsweise schwach im Fruchtcharakter.

**Beispiel 7:** Rahmenrezeptur für Weichspüler mit Meeresbrise-Charakter

| | |
|---|---|
| Boisambrene forte (Cognis) | 210 Teile |
| Timberol (Dragoco) | 20 Teile |
| Lilial (Givaudan) | 120 Teile |
| alpha-Hexylzimtaldehyd | 80 Teile |
| Floramat (Cognis) | 20 Teile |
| Lyral | 40 Teile |
| Sandelice (Cognis) | 10 Teile |
| Cyclovertal (Cognis) | 15 Teile |
| Anthoxan (Cognis) | 25 Teile |
| Dihydroisojasmonat (PFW) | 50 Teile |
| Ethyllinalool (Givaudan) | 60 Teile |
| Dimetol | 10 Teile |
| Troenan (Cognis) | 30 Teile |
| Timberol (Dragoco) | 20 Teile |
| Citronellol | 40 Teile |
| Geranylnitril (BASF) | 40 Teile |
| Ultralid (PFW) | 30 Teile |
| Galaxolid 50%ig (IFF) | 100 Teile |
| 2,5-Dimethyl-5-propyl-2-(2,4-dimethylphenyl)-1,3-dioxan, Bsp. 5 bzw DPG | 40 Teile |
| Cyclogalbanat (Dragoco) | 5 Teile |
| Ambroxan (Cognis) | 10 Teile |
| gamma -Decalacton | 5 Teile |
| Eugenol | 5 Teile |
| Melafleur (IFF) | 10 Teile |
| Calone (Pfizer) | 5 Teile |
| | 1000 Teile |

Der Zusatz von 40 Teilen der Verbindung gemäß Beispiel 5 zu der Mischung bereichert den Akkord mit einer leicht animalischen, den Algencharakter der Komposition verstärkenden Note. Die Vergleichsmischung mit 40 Teilen DPG im Austausch gegen die Verbindung gemäß Beispiel 5 zeigt dagegen einen mehr blumigen Charakter, der an der Wäsche nicht mehr so frisch erscheint.

## Patentansprüche

1. Verbindungen der allgemeinen Formel (I)

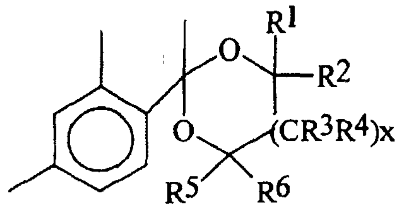

(I)

worin die Reste $R^1$ bis $R^6$ unabhängig voneinander Wasserstoff, Methyl, Ethyl, n-Propyl oder i-Propyl und x die Zahl 0 oder 1 bedeuten, mit der Maßgabe, dass diejenige Verbindung (I), bei der x = 0, $R^1$ = H, $R^5$ = H, $R^6$ = Methyl, $R^2$ = Methyl bedeutet, ausgenommen ist.

**2.** Verwendung von Verbindungen der allgemeinen Formel (I)

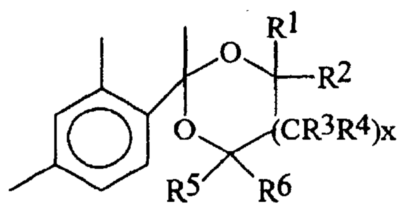

(I)

worin die Reste $R^1$ bis $R^6$ unabhängig voneinander Wasserstoff, Methyl, Ethyl, n-Propyl oder i-Propyl und x die Zahl 0 oder 1 bedeuten, als Riechstoffe.

**3.** Riechstoff-Kompositionen mit einem Gehalt an einem oder mehreren Verbindungen (I) gemäß Anspruch 1, wobei die Verbindungen (I) in den Kompositionen in einer Menge von 1 bis 70 Gew.-% - bezogen auf die gesamte Komposition - enthalten sind.

**Claims**

**1.** Compounds of the general formula (I)

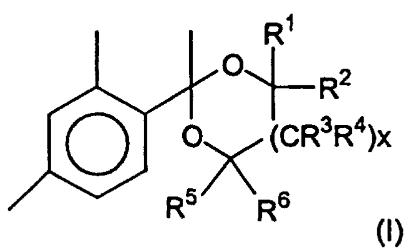

(I)

in which the residues $R^1$ to $R^6$ independently of one another represent hydrogen, methyl, ethyl, n-propyl or i-propyl and x is the number 0 or 1, with the proviso that that compound (I) is excluded in which x = 0, $R^1$ = H, $R^5$ = H, $R^6$ = methyl, $R^2$ = methyl.

**2.** The use of compounds of the general formula (I)

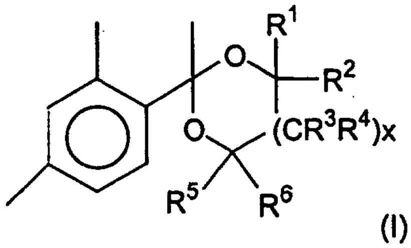

(I)

in which the residues $R^1$ to $R^6$ independently of one another represent hydrogen, methyl, ethyl, n-propyl or i-propyl and x is the number 0 or 1, as perfumes.

**3.** Perfume compositions containing one or more of the compounds (I) according to claim 1, the compounds (I) being present in the compositions in a quantity of 1 to 70% by weight, based on the composition as a whole.

**Revendications**

**1.** Composés de formule générale (I)

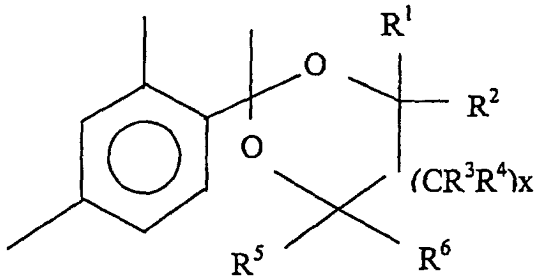

(I)

dans laquelle les restes $R^1$ à $R^6$ indépendamment les uns des autres, signifient de l'hydrogène, un méthyle, un éthyle, un n-propyle ou un i-propyle et x le nombre 0 ou 1, avec la restriction que le composé respectif (I) pour lequel x =0, $R^1$=H, $R^5$=H, $R^6$= méthyle, $R^2$= méthyle, est exclus.

**2.** Utilisation de composés de formule générale (I)

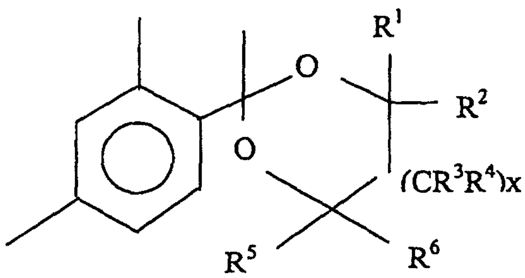

(I)

dans laquelle les restes $R^1$ à $R^6$indépendamment les uns des autres, signifient de l'hydrogène, un méthyle, un éthyle, un n-propyle ou un i-propyle et x le nombre 0 ou 1,
en tant que substances odoriférantes.

**3.** Compositions de substances odoriférantes ayant une teneur en un ou plusieurs composés (I) conformément à la

revendication 1, dans lesquelles les composés I sont présents dans les compositions en une quantité allant de 1 à 70 % en poids rapporté à la composition totale.